(19) 

Europäisches Patentamt

European Patent Office

Office européen des brevets

(11) **EP 3 474 864 B1**

(12) **EUROPEAN PATENT SPECIFICATION**

(45) Date of publication and mention
of the grant of the patent:
**02.04.2025 Bulletin 2025/14**

(21) Application number: **17749521.5**

(22) Date of filing: **28.06.2017**

(51) International Patent Classification (IPC):
*A61K 31/23* (2006.01)  *A61P 31/04* (2006.01)
*A61K 9/00* (2006.01)  *A61K 31/341* (2006.01)
*A61K 31/722* (2006.01)  *A61K 9/51* (2006.01)
*A61K 9/107* (2006.01)

(52) Cooperative Patent Classification (CPC):
(C-Sets available)
**A61K 31/722; A61K 9/0053; A61K 9/0065;
A61K 9/1075; A61K 9/5123; A61K 9/5161;
A61K 31/23; A61K 31/341; A61P 31/04**  (Cont.)

(86) International application number:
**PCT/IB2017/053885**

(87) International publication number:
**WO 2018/002853 (04.01.2018 Gazette 2018/01)**

(54) **NANOSTRUCTURED LIPID PARTICLES FOR USE IN THE TREATMENT OF DISEASES INDUCED BY HELIOBACTER PYLORI INFECTIONS**

NANOSTRUKTURIERTE LIPIDPARTIKEL ZUR VERWENDUNG BEI DER BEHANDLUNG VON DURCH HELIOBACTER PYLORI  INFEKTIONEN INDUZIERTEN KRANKHEITEN

PARTICULES LIPIDIQUES NANOSTRUCTURÉES DESTINÉES À ÊTRE UTILISÉES DANS LE TRAITEMENT DE MALADIES INDUITES PAR DES INFECTIONS PAR HELIOBACTER PYLORI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.06.2016  PT 2016109499**

(43) Date of publication of application:
**01.05.2019  Bulletin 2019/18**

(73) Proprietors:
• **INEB-Instituto Nacional De Engenharia
Biomédica
4200-135 Porto (PT)**
• **Universidade Do Porto
4099-002 Porto (PT)**

(72) Inventors:
• **LEAL SEABRA, Catarina
4200-135 Porto (PT)**
• **OLIVEIRA DE LACERDA NUNES PINHO, Cláudia
Daniela
4050-313 Porto (PT)**

• **DE LA SALETTE DE FREITAS FERNANDES
HIPÓLITO REIS
DIAS RODRIGUES, Maria
4050-313 Porto (PT)**
• **TEIXEIRA LOPES DA COSTA PINTO LOPES
MARTINS, Maria
Cristina
4200-135 Porto (PT)**

(74) Representative: **Patentree
Edificio Net
Rua de Salazares, 842
4149-002 Porto (PT)**

(56) References cited:
**WO-A2-2011/116963**

**(Cont. next page)**

• BELOQUI ANA ET AL: "Dextran-protamine coated nanostructured lipid carriers as mucus-penetrating nanoparticles for lipophilic d", INTERNATIONAL JOURNAL OF PHARMACEUTICS, ELSEVIER, NL, vol. 468, no. 1, 16 April 2014 (2014-04-16), pages 105 - 111, XP029029814, ISSN: 0378-5173, DOI: 10.1016/J.IJPHARM.2014.04.027

• LIN Y H ET AL: "Development of pH-responsive chitosan/heparin nanoparticles for stomach-specific anti-Helicobacter pylori therapy", BIOMATERIALS, ELSEVIER SCIENCE PUBLISHERS BV., BARKING, GB, vol. 30, no. 19, 1 July 2009 (2009-07-01), pages 3332 - 3342, XP026130550, ISSN: 0142-9612, [retrieved on 20090318], DOI: 10.1016/J.BIOMATERIALS.2009.02.036

• INÊS C GONÇALVES ET AL: "The potential utility of chitosan micro/nanoparticles in the treatment of gastric infection", EXPERT REVIEW OF ANTI-INFECTIVE THERAPY, vol. 12, no. 8, 11 August 2014 (2014-08-11), GB, pages 981 - 992, XP055414043, ISSN: 1478-7210, DOI: 10.1586/14787210.2014.930663

• ZAZO HINOJAL ET AL: "Current applications of nanoparticles in infectious diseases", JOURNAL OF CONTROLLED RELEASE, ELSEVIER, AMSTERDAM, NL, vol. 224, 6 January 2016 (2016-01-06), pages 86 - 102, XP029411481, ISSN: 0168-3659, DOI: 10.1016/J.JCONREL.2016.01.008

• SZNITOWSKA MALGORZATA ET AL: "The effect of a lipid composition and a surfactant on the characteristics of the solid lipid microspheres and nanospheres (SLM and SLN)", EUROPEAN JOURNAL OF PHARMACEUTICS AND BIOPHARMACEUTICS, vol. 110, 1 November 2016 (2016-11-01), pages 24 - 30, XP029858143, ISSN: 0939-6411, DOI: 10.1016/J.EJPB.2016.10.023

• SURANAN ANANTACHAISILP ET AL: "Chemical and structural investigation of lipid nanoparticles: drug-lipid interaction and molecular distribution", NANOTECHNOLOGY, IOP, BRISTOL, GB, vol. 21, no. 12, 26 March 2010 (2010-03-26), pages 125102, XP020174685, ISSN: 0957-4484

• CATARINA LEAL SEABRA ET AL: "Docosahexaenoic acid loaded lipid nanoparticles with bactericidal activity against Helicobacter pylori", INTERNATIONAL JOURNAL OF PHARMACEUTICS, vol. 519, no. 1-2, 11 January 2017 (2017-01-11), AMSTERDAM, NL, pages 128 - 137, XP055414008, ISSN: 0378-5173, DOI: 10.1016/j.ijpharm.2017.01.014

(52) Cooperative Patent Classification (CPC): (Cont.)

C-Sets
A61K 31/23, A61K 2300/00;
A61K 31/341, A61K 2300/00;
A61K 31/722, A61K 2300/00

## Description

### Technical field

The present disclosure relates to new nanostructured lipid carriers (NLCs - particles of imperfect crystal type, solid amorphous type (non-crystalline matrix) or multiple type) that are effective treatment for use in the treatment of disease induced by Helicobacter pylori.

The present disclosure is also directed to compositions that comprise such nanoparticles and to a method of their production.

### Background

*Helicobacter pylori* is a Gram-negative bacteria that infect one-half of the World population. This bacterium is the major etiological factor in chronic active gastritis, gastric ulcers, and gastric cancer [1-3].

The currently recommended treatment includes a combination of two antibiotics, commonly clarithromycin plus amoxicillin or metronidazole, and a protonpump inhibitor [4]. However, this therapy fails in 20% of patients for several reasons, but principally due to poor patient compliance and bacteria resistance to the antibiotics used [5]. More complex regimens, including the use of non-bismuth or bismuth-containing quadruple therapies, were also recommended as a second-line option [4], but their complexity potentiates patient non-compliance and bacterial resistance, leading to treatment failure. The exacerbated increase of antibiotic resistance has generated alarming impact and new antibiotic-free strategies are necessary. Gonçalves et al., Expert review of anti-infective therapy, vol. 12, no. 8, 2014, discloses the use of chitosan for the treatment of H. pylori infections.

The application of nanotechnology, especially, the use of nanoparticles as drug nanocarriers has generated a significant impact in medicine. The increasing interest by lipid nanocarriers is associated with their higher biocompatibility and lower toxicity compared to polymeric nanoparticles, as well as lower production cost and scalability [6-9]. Nanostructured lipid carriers (NLCs) are lipid nanoparticles specifically designed and patented as delivery systems for pharmaceutical, cosmetic and/or alimentary active ingredients [10]. They are characterized by a solid lipid core consisting of a mixture of solid and liquid lipids. The resulting particles matrix displays a melting depression when compared with the original solid lipid but is still solid at body temperature [6, 7]. NLCs can be prepared using a wide variety of lipids including fatty acids, glyceride mixtures or waxes, stabilized with selected biocompatible surfactants (non-ionic or ionic). Moreover, most of NLCs ingredients are safe and under the Generally Recognized as Safe (GRAS) status issued by the Food and Drug Administration (FDA) [11, 12]. Using spatially different lipids induces many imperfections in the crystal and if a proper amount of liquid lipid is mixed with the solid lipid, a phase separation and the formation of oily nanocompartments within the solid lipid matrix can occur [13].

This invention describes the specific bactericidal activity of NLCs without any active ingredient (unloaded-NLC), prepared using glyceryl palmitostearate as solid lipid, caprylic/capric triglyceride as liquid lipid and polysorbate 60 as a surfactant against *Helicobacter pylori.* This effect was not observed against other bacteria, such as the Gram-positive *Staphylococcus epidermidis* and *Lactobacillus casei* and the Gram-negative *Escherichia coli.* The behavior of unloaded-NLCs against *H. pylori* was not expected, since results described in the literature, that only uses unloaded-NLCs as controls of the drug-loaded NLCs, always demonstrated their null or very low activity against other bacteria [14, 15]. Moreover, the utilization of NLCs, with and without drugs, for the treatment of *H. pylori* gastric infection was not previously reported.

These facts are disclosed in order to illustrate the technical problem addressed by the present disclosure.

### General Description

The present disclosure relates to new nanostructured lipid carriers (NLCs - particles of imperfect crystal type, solid amorphous type (non-crystalline matrix) or multiple type) that are effective treatment for use in the treatment of disease induced by Helicobacter pylori. The present disclosure is also directed to compositions and to a method of their production. The application also describes compositions in which these compounds are used.

The present disclosure relates to the bactericidal activity of nanostructured lipid carriers (NLCs - particles of imperfect crystal type, solid amorphous type (non-crystalline matrix) or multiple type), without carrying pharmacological agents, the characterization of said antibacterial NLCs, and their use as an agent to kill Helicobacter pylori.

Surprisingly these NLCs do not affect other bacteria, such Lactobacillus casei, Staphylococcus epidermidis and Escherichia coli. Moreover, these NLCs are not cytotoxic to gastric cells at bactericidal concentrations. This invention opens new perspectives for the development of an antibiotic-free treatment against H. pylori gastric infection.

An aspect of the present disclosure is relate to a nanostructured lipid particle for use in the treatment or prevention of a disease induced by Helicobacter pylori comprising

a mixture of a solid lipid, a liquid lipid, a surfactant and a solvent;
wherein the solid lipid is glyceryl palmitostearate,;
wherein the liquid lipid is Caprylic/Capric Triglyceride;
wherein the surfactant is polysorbate 60;
wherein the nanostructured lipid particle is a Helicobacter pylori bactericide.

The nanostructured lipid particle comprises particles of imperfect crystal type, a solid amorphous type (non-crystalline matrix) or multiple type, and wherein said nanoparticles are capable of killing Helicobacter pylori.

In an embodiment, the nanostructured lipid may comprises

30-65 % (wt/v) of solid lipid;
10-30 % (wt/v) the liquid lipid;
5-20% (wt/v) a surfactant.

In an embodiment, the nanostructured lipid may comprises

52 % (wt/v) of solid lipid;
23 % (wt/v) the liquid lipid;
15 % (wt/v) a surfactant.

In an embodiment, solvent is water.

In an embodiment, the particles can be produced by mixture of several lipids forming a nanoemulsion, using techniques that may include the use of hot homogenization and ultrasonication.

In an embodiment, the particles may comprise diameters is between 50 and 400 nanometers, preferably between 150 and 250 nanometers, as determined by their hydrodynamic size using dynamic light scattering, at 37 °C.

In an example, the nanostructured lipid particle may be use in the treatment or prevention of peptic ulcer, gastrite, gastric neoplasia; in particular intestinal metaplasia or gastric cancer.

In an embodiment, the nanostructured lipid can be stored during 1 month, in aqueous suspension at 4° - 20 °C.

Another aspect of the present disclosure relates to an oral composition for use in the treatment or prevention of a disease induced by Helicobacter pylori comprising the nanostructured lipid particle in the present disclosure in a therapeutically effective amount. In particular an oral composition.

In an embodiment, the oral composition may further comprise at least one therapeutically active substance selected from the group of consisting of a gastrointestinal protectant or an antibiotic, or mixtures thereof.

In an embodiment, the gastrointestinal protectants is a proton pump inhibitors, in particular an imidazole compound.

In an embodiment, the antibiotic is selected from a group consisting of: amoxicillin, clarithromycin, metronidazole, tetracycline, or mixtures thereof.

In an embodiment, the composition may further comprise gastric retentive polymer. Preferably the gastric retentive polymer is selected from a list consisting of polyalkylene oxides, such as polyethylene glycols, particularly high molecular weight polyethylene glycols; cellulose polymers and their derivatives including, but not limited to, hydroxyalkyl celluloses, hydroxymethyl cellulose, hydroxyethyl cellulose, hydroxypropyl cellulose, hydroxypropylmethyl cellulose, carboxymethylcellulose, microcrystalline cellulose; polysaccharides and their derivatives; chitosan; poly(vinyl alcohol); xanthan gum; maleic anhydride copolymers; poly(vinyl pyrrolidone); starch and starch-based polymers; maltodextrins; poly (2-ethyl-2-oxazoline); poly(ethyleneimine); polyurethane; hydrogels; crosslinked polyacrylic acids, or combinations thereof; in particular chitosan.

An aspect of the present disclosure is relate to a nanostructured lipid particle comprising :particles of imperfect crystal type, a solid amorphous type (non-crystalline matrix) or multiple type, and wherein said nanoparticles are capable of killing Helicobacter pylori.

In an embodiment, the nanostructured lipid particle of the present disclosure wherein the particles can be produced by mixture of several lipids forming a nanoemulsion, using techniques that may include the use of hot homogenization and ultrasonication.

In an embodiment, the nanostructured lipid particle of the present disclosure, wherein the particles present with diameters between 50 and 400 nanometers, preferably between 150 and 250 nanometers, as determined by their hydrodynamic size using dynamic light scattering, at 37°C.

In an embodiment, the nanostructured lipid particle of the present disclosure wherein the particle is formed by mixtures of different lipids, selected from solid and liquid lipids and mixed with a surfactant to produce a nanoemulsion. The lipids are glyceryl palmitostearate, and Caprylic/Capric Triglyceride and, the surfactant is polysorbate 60.

In an embodiment, the nanostructured lipid particle of the present disclosure, wherein the therapeutically effective amount of Helicobacter pylori infection is administrated orally.

In an embodiment, the nanostructured lipid particle of the present disclosure can be stored during 1 month, in aqueous suspension at 4 ° and 20 °C.

In an embodiment, the nanostructured lipid particle of the present disclosure wherein the therapeutically effective amount against Helicobacter pylori does not affect other bacteria, particularly Lactobacillus casei, Staphylococcus epidermidis and Escherichia coli.

In an embodiment, the nanostructured lipid particle of the present disclosure, in combination with one or more therapeutically active substance(s) selected from the group of gastrointestinal protectants (particularly proton pump inhibitors) and antibiotics (particularly amoxicillin, clarithromycin, metronidazole, tetracycline) used in the treatment of Helicobacter pylori infection.

Another aspect of the present disclosure relates to an oral formulation containing the nanostructured lipid particles of the present disclosure, wherein the dosage form can be administrated in any formulation adapted for stomach delivery, particularly incorporated/combined with gastric retentive polymers such as chitosan or other pharmaceutical compounds able to release the particles in the stomach.

Brief Description of the Drawings

The following figures provide preferred embodiments for illustrating the description and should not be seen as limiting the scope of invention.

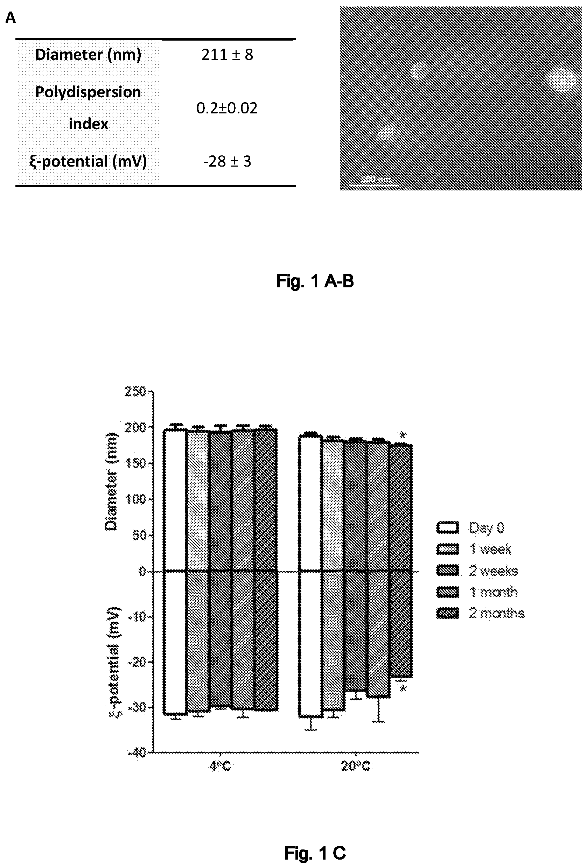

**Figure 1:** Schematic representation of NLCs characterization in terms of (A) diameter (bars), polydispersion index and charge surface ($\xi$-potential) performed using dynamic light scattering at 37°C, (B) morphology evaluated by cryo-scanning electron microscopy (Cryo-SEM) (scale bar 500nm, 50000x magnification) and (C) effect of time of storage in water at 4°C and 20°C on diameter and charge ($\xi$-potential) of unloaded-NLC produced. Data is reported as the mean $\pm$ standard deviation (n=3). *$p < 0.05$ compared to different time-points on day 0 (Kruskal-Wallis test).

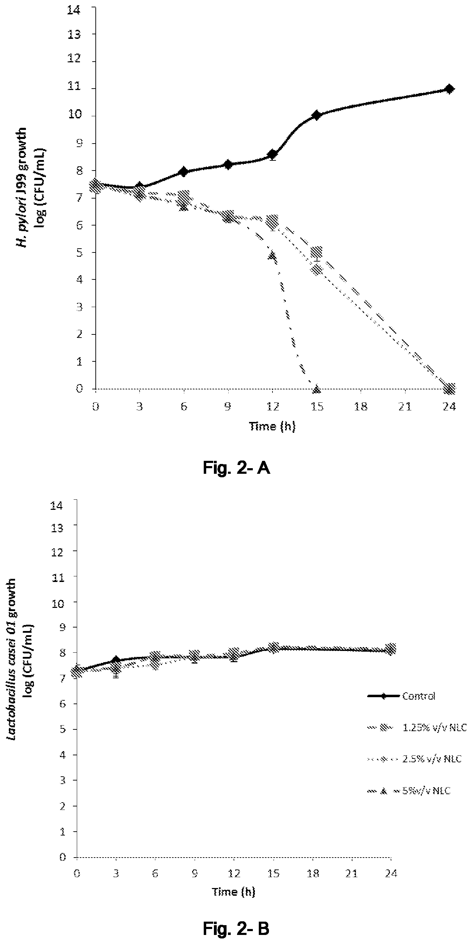

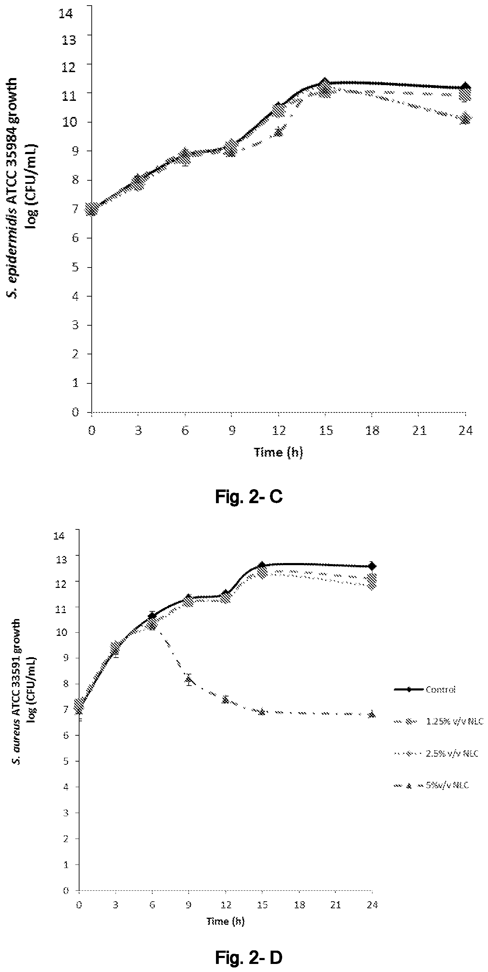

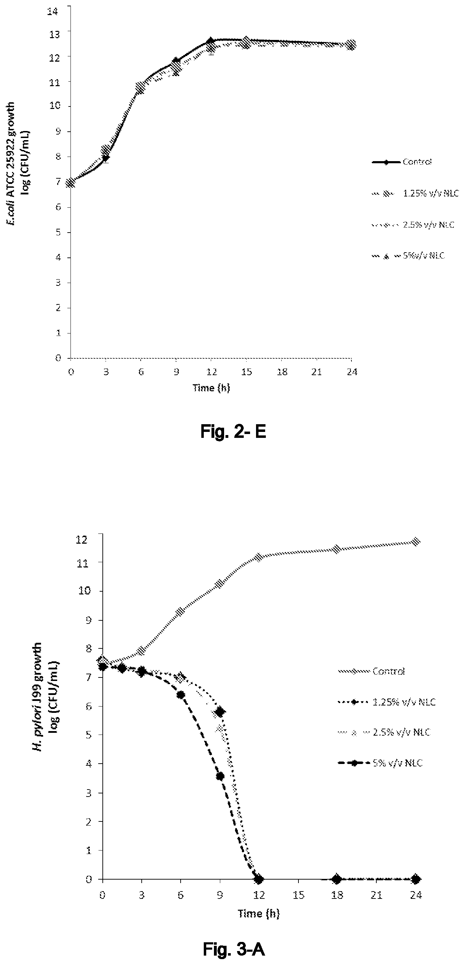

**Figure 2:** Schematic representation of the growth of bacteria in increasing concentrations of unloaded-NLC in Müller-Hinton Broth medium supplemented with 10% FBS: (A) *H. pylori* J99, (B) *Lactobacillus casei-01, (C) Staphylococcus epidermidis* ATCC35984, (D) *Staphylococcus aureus* ATCC 33591 (methicillin resistant) and *Escherichia coli* ATCC 25922. Numerical data is reported as mean $\pm$ standard deviation (n=3). All concentrations tested with unloaded-NLCs were significantly different compared to each bacteria growth treated with unloaded-NLCs and bacteria untreated (control) ($p < 0.05$, Kruskal-Wallis test).

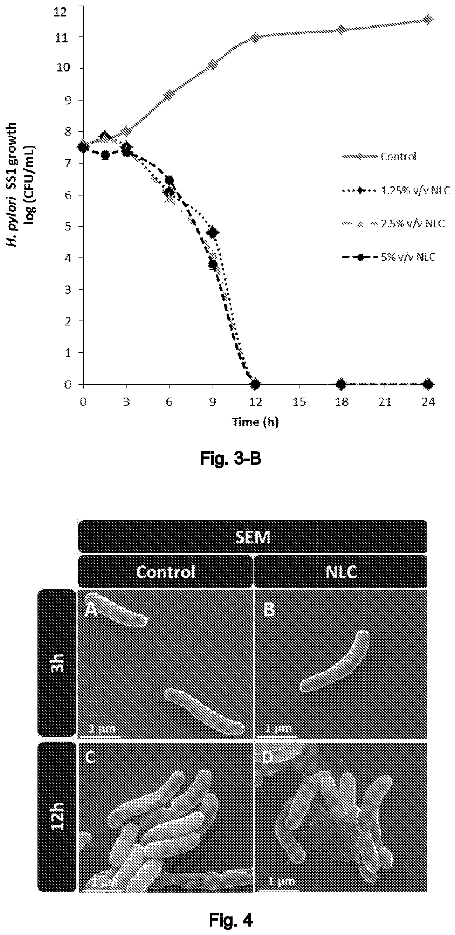

**Figure 3.** Schematic representation of the growth of (A) *H. pylori* J99 and (B) *H. pylori* SS1 in increasing concentrations of unloaded-NLC in Brucella Broth medium supplemented with 10% FBS. Numerical data is reported as mean $\pm$ standard deviation (n=3). All concentrations tested with unloaded-NLCs were significantly different compared to each bacteria growth treated with unloaded-NLCs and bacteria untreated (control) ($p < 0.05$, Kruskal-Wallis test).

**Figure 4.** Scanning Electron micrographs of *H. pylori* J99 incubated during 3h (A to B) and 12h (C to D) with unloaded-NLC. In A and C, SEM images of *H. pylori* J99 was not treated (control); in B and D, *H. pylori* J99 was treated with 1.25 %v/v of unloaded-NLC. The scale bar in the SEM image represents 1 $\mu$m, 60 000 x magnifications.

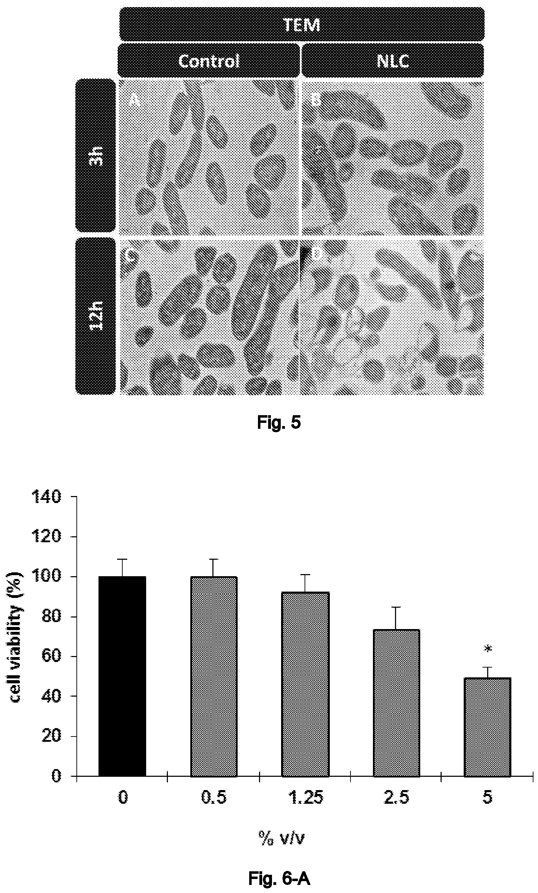

**Figure 5.** Transmission Electron micrographs of *H. pylori* J99 incubated during 3h (A to B) and 12h (C to D) with unloaded-NLC. In A and C, TEM images of *H. pylori* J99 was not treated (control); in B and D, *H. pylori* J99 was treated with 1.25%v/v of unloaded-NLC. The scale bar in the TEM image represents 200 nm, 50 000 $\times$ magnifications.

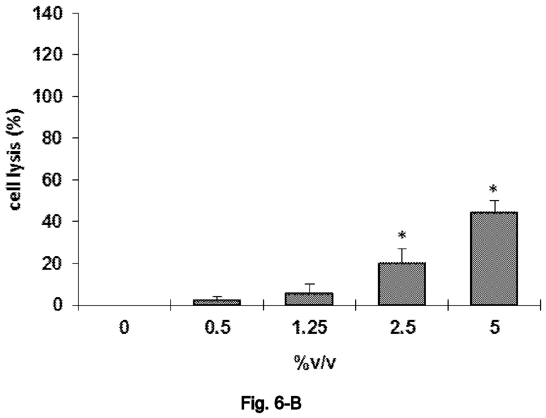

**Figure 6.** Effect of unloaded-NLCs of gastric carcinoma cell line in increasing amounts. MKN45 gastric carcinoma (A) cell line viability and (B) cytotoxicity assessed by the MTT and LDH assay, respectively, after 24h exposure to different amounts of NLCs. Data is reported as mean $\pm$ standard deviation (n=3). * $p < 0.05$, refers to significant differences of MKN45 gastric cells treated cells with unloaded-NLC and cells untreated (control) (Kruskal-Wallis test).

Detailed Description

This invention describes the unexpected specific bactericidal effect of unloaded-NLCs (Nanostructured Lipid Carriers) on *H. pylori* without affecting other bacteria, such as the Gram-positive *Staphylococcus epidermidis* and *Lactobacillus casei,* and the Gram-negative *Escherichia coli.* NLCs were developed to be used in the preparation of pharmaceutical, cosmetics and/or alimentary compositions as delivery systems for an active ingredient. Therefore, their bactericidal effect per sees (without any active ingredient/drug) was not expected and was not previously described.

In an embodiment, nanostructured Lipid Carriers (NLCs) were produced using 200 mg of glyceryl palmitostearate (Gattefosé) as solid lipid, 90 mg of Caprylic/Capric Triglyceride (Acofarma) as liquid lipid and 60 mg of polysorbate 60 (Merck) as a surfactant. NLCs were produced using hot homogenization and ultrasonication technique. Briefly, lipids (solid and liquid) and surfactant were weighted together and heated at a temperature above the solid lipid melting point to promote their mixture. A nanoemulsion was obtained after adding to the lipid mixture, Milli-Q water preheated above the solid lipid melting point under high speed stirring (12000 rpm, 20s) using an ultra-turrax T25 (Janke and Kunkel IKA-Labortechnik). This microemulsion was homogenized using a sonicator (Vibra-Cell model VCX 130 equipped with a VC 18 probe, Sonics and Materials Inc., Newtown), with tip diameter ¼" (6 mm), at 60% amplitude for 5 minutes. Nanoemulsion was cooled to room temperature under gentle magnetic stirring allowing the inner phase of NLCs to solidify forming nanoparticles dispersed in the aqueous phase without aggregates.

In an embodiment, the hydrodynamic size distribution and surface charge ($\xi$-potential) of NLCs were characterized by dynamic light scattering (DLS) using the Malvern Zetasizer Nano ZS. Clear disposable folded capillary cells (DTS1070) from Malvern were used for all samples. Samples were diluted (1:50) with Milli-Q water conducted at a backscattering angle of 173° at 37°C. All measurements were performed in triplicate.

In an embodiment, the morphology of NLC was observed by Cryo-Scanning Electron Microscopy (CryoSEM) using a JEOL JSM-6301F, an Oxford Instruments INCA Energy 350 and a Gatan Alto 2500. A drop of nanoparticles was placed on a grid, rapidly cooled in a liquid nitrogen slush (-210°C), and transferred under vacuum to the cold stage of the preparation chamber. The sample was fractured, sublimated for 120 seconds min at -90°C to reveal greater detail, and coated with a gold-palladium alloy by sputtering for 40 seconds. Finally, the sample was then transferred under vacuum into the CryoSEM chamber where they were observed at -150°C.

In an embodiment, NLCs were storage in Milli-Q water during 2 months at two different temperatures: 4°C and 20°C. Nanoparticles stability was evaluated by periodic measurements of their size and surface charge using the Malvern Zetasizer Nano ZS, as described above. All measurements were performed in triplicate.

In an embodiment, microorganisms used were the following: Helicobacter pylori J99 (H. pylori; obtained from the Department of Medical Biochemistry and Biophysics, Umea University, Sweden [16]), mouse-adapted H. pylori strain SS1 (obtained from Unité de Pathogenèse de Helicobacter, Institute Pasteur, France), Lactobacillus casei-01 (Chr. Hansen, Hørsholm Denmark) (L. casei), Staphylococcus epidermis ATCC 35984 (S. epidermidis), Staphylococcus aureus ATCC 33591 (methicillin resistant) (S. aureus MRSA) and Escherichia coli ATCC 25922 (E. coli). Each bacterium was grown on specific solid medium plates and overnight on a liquid medium, as described in Table 1.

Table 1- Specific solid and liquid medium of each bacteria used in this study.

| Microorganism | Solid medium | Liquid medium |
|---|---|---|
| *H. pylori* J99 and *H. pylori* SS1 | Blood agar base 2 (Oxoid) plates supplemented with 10% defibrinated horse blood (Probiológica) and with an antibiotics-cocktail of 0.155g/L Polymixine B (Sigma-Aldrich), 6.25 g/L Vancomycin (Sigma-Aldrich), 1.25g/L Amphotericin B (Sigma-Aldrich) and 3.125g/L Trimethroprim (Sigma-Aldrich) | Brucella Broth (BB, Oxoid) supplemented with 10% of Fetal Bovine Serum (FBS, Gibco) |
| *L. casei* 01 | De Man-Rogosa and Sharpe agar (MRS agar, Biokar) | De Man-Rogosa and Sharpe broth (MRS broth, Biokar) |
| *S. epidermidis* ATCC 35984 *S. aureus* ATCC 33591 *E. coli* ATCC 25922 | Tryptic Soy Agar (TSA, Merck Millipore) | Tryptic Soy Broth (TSB, Merck Millipore) |

In an embodiment, bacteria were pre-cultured on specific liquid medium (Table1) overnight at 37°C and 150 rpm. After washing with phosphate buffered saline (PBS 1x, pH 7.4), bacteria were adjusted to approximately $1 \times 10^7$ CFU/mL in Müeller-Hinton broth (MHB, Merck Millipore) supplemented with 10% of FBS. *H. pylori* was also tested in BB+10% FBS. Different NLC concentrations (0%, 1.25%, 2.5% and 5% v/v) were added to bacteria culture and they're incubated during 24h, at 37°C, 150 rpm, and in the case of *H. pylori* under microaerophilic conditions. At different time-points (0, 3, 6, 9, 12, 15, 24h), a 200 $\mu$L sample of each bacterial culture was isolated, serially diluted, plated on solid medium plates (Table 1) and incubated at 37°C for 5 days for *H. pylori,* 2 days for *L. casei* and 24h for the other bacteria. The number of viable bacteria per mL was determined by colonies forming unit (CFU) counting.

In an embodiment, Morphological changes of H. pylori J99 were studied by SEM, analyzing the effect of unloaded-NLCs on bacteria. Bacteria were incubated with unloaded-NLC (1.25% v/v) for 3 and 12h as previously described using BB medium supplemented with 10%FBS. After incubation, samples were washed in phosphate buffered saline (PBS 1x, pH 7.4) and fixed in 2.5% glutaraldehyde (Merck) in 0.14M sodium cacodylate buffer (Merck) for 30 min at room temperature. Fixed bacteria adhered on glass coverslips in a 24-well suspension plate during 2h at room temperature. Then samples were dehydrated with an increasing ethanol/water gradient (50% v/v to 99% v/v) and subjected to critical point drying (CPD 7501, Poloran). Finally, the samples were sputter-coated with gold/palladium film over 30s. H. pylori samples were observed by scanning electron microscopy (SEM; JEOL JSM-6310F), at magnification 30000 and 60000x, at the CEMUP (Centro de Materiais da Universidade do Porto).

In an embodiment, the effect of unloaded-NLCs on the structure of H. pylori J99 was also analyzed by TEM. Incubation time and concentrations were similar as described for SEM analysis. After incubation, bacteria suspension was centrifuged (3000g, 5min) and the supernatant removed. The bacterial pellet was fixed by resuspending in a mixture of 4% w/v of paraformaldehyde (Merck) with 2.5% v/v glutaraldehyde (Electron Microscopy Sciences) in 0.14M sodium cacodylate buffer (Merck) (pH 7.4). Samples were then washed with sodium cacodylate buffer, centrifuged and bacterial pellet post-fixed in 2% osmium tetroxide (Electron Microscopy Sciences) in sodium cacodylate buffer was embedded in a HistoGel (Thermo Scientific) and processed in Epon resin (Electron Microscopy Sciences). Ultrathin sections of 50 nm thickness were performed on a Ultramicrotome (RMC PowerTome PC model), by using diamond knives (Diatome). Sections were mounted on formvar-coated nickel grids, stained with uranyl acetate and lead citrate (Delta Microscopies) and examined using TEM (JEOL JEM 1400 transmission electron microscope) equipped with a CCD digital camera Orious 1100W at i3S (Instituto de Investigaçao e Inovação em Saúde da Universidade do Porto).

In an embodiment, was measure the effect of nanostructured lipid carriers on gastric cells line, gastric carcinoma cell line MKN45 was grown in RPMI 1640 with Glutamax and HEPES (Gibco) supplemented with 10% inactivated FBS (Gibco) and 1% penicillin-streptomycin (Gibco) at 37°C under 5% CO2 in humidified air. The medium was changed every two days. For subculturing, cells were trypsinized and counted in a Neubauer chamber diluted (1:10) in a Trypan Blue solution (0.4% w/v) (Sigma-Aldrich). Cells were expanded in 75 cm2 T-flasks with appropriate aliquots of the cell suspension with a sub-cultivation ratio of 1:3. NLC effect on MKN45 gastric cells was evaluated using direct contact assay. MKN45 cells were seeded in a 12-well plate (5×105 cell per well) in RPMI 1640 with Glutamax and HEPES supplemented with 10% inactivated FBS and 1% PenStrep, at 37°C and under 5% CO2 in humidified air, during 48h. Then, the culture medium was changed and NLCs were added with different concentrations (0, 0.5%, 1.25%, 2.5%, 5.0% v/v). Well-plate was incubated at 37°C and under 5% CO2 in humidified air, during 24h. Cells in TritonTM X-100 (2% w/v) (Sigma-Aldrich) and cells in fresh culture medium were used as a control, in order to normalize the results. After 24h, the supernatant was removed (transferred to 12-well plates and stored for cytotoxicity assay) and 1 mL of 5 mg/mL of Thiazolyl Blue Tetrazolium Bromide (MTT, 98%, Sigma-Aldrich) in PBS solution was added to the cultures, diluted to a final concentration of 0.5 mg/L in culture medium and incubated for 3h at 37°C in the dark. Then, MTT solution was discarded and 1 mL of DMSO was added, to solubilize the formazan crystals formed by MTT reaction. The plate was shaken for 5 min, at room temperature, under light protection and then, the optical density was measured at 590 nm and 630 nm using a microplate reader (Synergy™ H Multi-mode Microplate Reader, BioTek Instruments). MTT assay evaluates the activity of cellular oxidoreductase enzymes inside mitochondria by converting the MTT tetrazolium dye into its insoluble formazan (purple) that is directly proportional to the number of viable cells. The percentage of cell viability was calculated according to the following equation:

$$\text{Cell viability (\%)} = \frac{\text{Experimental value} - \text{Triton control}}{\text{Medium control} - \text{Triton control}} \times 100$$

In an embodiment, Lactate dehydrogenase (LDH) is released to the cell culture medium when the cytoplasmic membrane is damaged. Consequently, its quantification allows having an assessment of cell death. Hence, the 12-well plates containing the cell culture supernatant collected for the MTT assay were centrifuged (250g, 10min at room temperature). Carefully, without disturbing the pellet, 100 µl of the sample were transferred to the 96-well plate and 100 µl of the LDH Cytotoxicity Detection Kit (Takara Bio Inc) were added. After 5 min of incubation, at room temperature and protected from light, the absorbance was measured at 490 and 630 nm using the microplate reader. The percentage of cytotoxicity was calculated according to the following equation:

$$\text{Cytotoxicity (\%)} = \frac{\text{Experimental value} - \text{Medium control}}{\text{Triton control} - \text{Medium control}} \times 100$$

In an embodiment, Data are reported as means ± standard deviation. Data from different groups were compared statistically using non-parametric Kruskal-Wallis test. Analyses were performed with a significance level of 0.05 using Graph Pad Prism 5.0 (Graph-Pad Software).

In an embodiment, NLCs were synthesized using a modified hot homogenization technique with no use of organic solvents [17]. Results obtained by DLS demonstrated that NLCs have a homogenous size distribution with a mean diameter of 211 ± 8 nm (Figure 1A). Polydispersion index was around 0.2 suggesting low size variability (monodisperse distribution) and no visible aggregation that was confirmed by Cryo-SEM. However, when observed by Cryo-SEM (Figure 1B), NLCs are slightly smaller, which could be related to the sublimation process used in this technique to remove the surface water that can also remove the water present in the nanoparticle matrix causing particle shrinkage. NLCs size can be important for *H. pylori* treatment since it was described that particles with sizes between 130 and 300 nm are able to infiltrate gastric cell-cell junctions and interact locally with *H. pylori* infection sites in intercellular spaces [18].

In an embodiment, ξ-potential is an important factor in the analysis of colloidal dispersions stability. Figure 1A also shows that NLCs have a ξ-potential around -28mV. According to literature, colloidal dispersions are stable when they are strongly charged (|130 mV|) (i.e., aggregation is avoided) [19], whereas ξ-potential values in the range of -10<0<+10mV are considered neutral [20]. Since ξ-potential is near -30mV, these NLCs can be considered physically stable.

In an embodiment, NLCs are stable during storage in aqueous suspension for at least 1 moth at 4° and 20°C in terms of size and charge (Figure 1C). However, at 20°C and after 1 month of storage, there is a slight increase of the ξ-potential from -27 to - 23 mV. Nevertheless, these negative charges are enough to maintain their physical stability and avoid nanoparticles aggregation.

In an embodiment, NLC bactericidal activity was evaluated following bacteria growth during 24h in the presence of different NLC concentrations (Figure 2) using Müller-Hinton Broth (MHB), which is the recommended medium for the determination of the minimal inhibitory concentration according to the guidelines proposed by two recognized organizations: CLSI (Clinical & **Laboratory Standards Institute)** and EUCAST (European Society of Clinical Microbiology and Infectious Diseases) [21]. However, since *H. pylori* is a fastidious microorganism and requires a complex nutrient-rich growth media, MHB had to be supplemented with 10% of fetal bovine serum (FBS). This supplement may act as an additional source of nutrient and also protect against the toxic effects of long-chain fatty acids [22]. Nevertheless, for comparison and to demonstrate the specific bactericidal activity of NLCs to *H. pylori,* this FBS-supplemented medium was also used for bactericidal assays performed with other bacteria.

In an embodiment, Minimal bactericidal concentration (MBC) was defined as the minimal drug concentration to kill 99.9% (>3 logs) bacteria in 24h of incubation. Figure 2A shows that NLCs are bactericidal against *H. pylori* at all concentrations used, since after 24h in the presence of NLCs, there are no live bacteria. For the highest concentration tested (5% v/v), *H. pylori* was killed after 15h of incubation time. Nonetheless, an inhibitory effect of 50% (> 1log) was detected after 9h of incubation with this NLC concentration.

In an embodiment, NLC bactericidal effect was not detected on *Lactobacillus casei-01* (Figure 2B), Gram-positive rod-shaped and nonpathogenic bacteria found on the human gut microbiota. This bacterium has an importance for gut microbiota which enhances the integrity of the intestinal barrier, decrease translocation of bacteria across the intestinal mucosa and disease phenotypes such as gastrointestinal infections [23, 24]. This specific bactericidal activity of unloaded-NLCs against *H. pylori* (without affecting *L. casei*) opens new routes for the treatment of *H. pylori* infection without affecting gut microbiota.

In an embodiment, NLCs were also tested on other Gram-positive cocci bacteria, *Staphylococcus epidermidis* (Figure 2C) and methicillin-resistant *Staphylococcus aureus* (MRSA) (Figure 2D). No bactericidal effect was observed for both bacteria on the concentrations used. However, the higher NLCs concentration (5% v/v) had an inhibitory effect on MRSA growth. Other bacterium tested was *Escherichia coli,* a Gram-negative rod-shaped bacterium, is the most prevalent commensal inhabitant of the human intestinal tract and lives in a mutually beneficial association with hosts [25, 26]. *E. coli* is not usually pathogenic. NLC bactericidal effect was not detected on *E. coli* (Figure 2E); therefore, unloaded-NLCs can be used without to affect the normal flora of the gut.

In an embodiment, the NLC bactericidal effect against *H. pylori* was also tested using a growth medium (BB) indicated for *H. pylori* growth [27]. Figure 3 shows that all bacteria are killed after 12h of incubation with NLCs for all the concentrations used. The bactericidal effect of NLC on *H. pylori* was faster when bacteria were growing in this medium than in MHB medium.

In an embodiment, in order to understand the interaction of NLCs with *H. pylori,* their morphology after growing in the presence and absence of NLCs was evaluated by SEM and TEM. Samples were prepared after 3 and 12h of incubation with 1.25% of NLCs, since after 3h mostly of the bacteria are alive and after 12h all bacteria are dead.

In an embodiment, SEM images of *H. pylori* grown during 3h (Figure 4A) and 12h (Figure 4C) in the absence of NLCs (control) show their characteristic bacillus shape with 2-4$\mu$m of length and 0.5-1$\mu$m wide [4-6]. When bacteria were incubated with NLCs for 3h (Figure 4B) their morphology was similar to the controls, demonstrating the integrity of its membrane. However, after 12h incubation with NLCs (Figure 4D), although high morphological changes were not observed using this technique, it was possible to visualize the leakage of their cytoplasmic contents. These results suggest that NLCs can interact with *H. pylori* by disrupting its cell membrane.

In an embodiment, TEM images (Figure 5) corroborate with SEM images, showing *H. pylori* with the intact cell membrane and dense cytoplasm in controls (Figure 5A and 5C) and after 3 hours growing with NLCs (Figure 5B). After 12h of incubation with NLCs, TEM images revealed a disseverance of outer membrane and plasma membrane with parts of bacteria without cytoplasmic contents (Figure 5D).

In an embodiment, NLCs cytotoxicity was evaluated against a gastric carcinoma cell line (MKN45) using thiazolyl blue tetrazolium bromide (MTT) and lactate dehydrogenase (LDH) assays (Figure 6A and 6B, respectively). Figure 6A shows that for NLC concentrations up to 2.5%v/v, the reduction of MKN45 cell viability was lower than 30%. Figure 6B demonstrated that despite its strong bactericidal activity against *H. pylori,* NLCs are not cytotoxic against MKN45 cells at concentrations up to 2.5%v/v (~20% cell lysis). These results also demonstrated that NLCs at bactericidal concentrations (1.25% v/v) have a negligible lactate dehydrogenase release and did not induce apoptosis and mitochondrial dysfunction after 24h of incubation. These NLCs, at bactericidal concentrations, are biocompatible according to ISO 10993-5 [12] since the decrease of cell metabolic activity and increase of cell lysis due to the presence of NLCs in comparison with control were lower than 30%.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## REFERENCES

1. Correa, P. and J. Houghton, Carcinogenesis of Helicobacter pylori. Gastroenterology, 2007. 133(2): p. 659-672.

2. Amieva, M. and R.M. Peek Jr, Pathobiology of Helicobacter pylori-Induced Gastric Cancer. Gastroenterology, 2016. 150(1): p. 64-78.

3. Matysiak-Budnik, T. and F. Mégraud, Helicobacter pylori infection and gastric cancer. European Journal of Cancer, 2006. 42(6): p. 708-716.

4. Malfertheiner, P., et al., Management of Helicobacter pylori infection-the Maastricht IV/ Florence Consensus Report. Gut, 2012. 61(5): p. 646-664.

5. Vakil, N., Helicobacter pylori treatment: a practical approach. Am J Gastroenterol, 2006. 101(3): p. 497-9.

6. Carbone, C., et al., Pharmaceutical and biomedical applications of lipid-based nanocarriers. Pharm Pat Anal, 2014. 3(2): p. 199-215.

7. Battaglia, L. and M. Gallarate, Lipid nanoparticles: state of the art, new preparation methods and challenges in drug delivery. Expert Opinion on Drug Delivery, 2012. 9(5): p. 497-508.

8. Martins, S., et al., Lipid-based colloidal carriers for peptide and protein deliveryliposomes versus lipid nanoparticles. Int J Nanomedicine, 2007. 2(4): p. 595-607.

9. Müller, R.H., K. Mäder, and S. Gohla, Solid lipid nanoparticles (SLN) for controlled drug delivery - a review of the state of the art. European Journal of Pharmaceutics and Biopharmaceutics, 2000. 50(1): p. 161-177.

10. Viladot Petit, J.L.L., G.R. Delgado, and B.A. Fernández, Lipid nanoparticle capsules, in US 2013/0017239 A1. 2011, LIPOTEC S.A.: Spain.

11. Mehnert, W. and K. Mäder, Solid lipid nanoparticles: Production, characterization and applications. Advanced Drug Delivery Reviews, 2001. 47(2-3): p. 165-196.

12. Svilenov, H.T., Christo Solid lipid nanoparticles - a promising drug delivery system, in Nanomedicine, A.M. Seifalian, Achala de Kalaskar, Deepak M., Editor. 2014, One Central Press (OCP): London.

13. Muchow, M., P. Maincent, and R.H. Müller, Lipid Nanoparticles with a Solid Matrix (SLN®, NLC®, LDC®) for Oral Drug Delivery. Drug Development and Industrial Pharmacy, 2008. 34(12): p. 1394-1405.

14. da Silva, P.B., et al., A Nanostructured Lipid System as a Strategy to Improve the in Vitro Antibacterial Activity of Copper(II) Complexes. Molecules, 2015. 20(12): p. 22534-45.

15. Manea, A.-M., B.S. Vasile, and A. Meghea, Antioxidant and antimicrobial activities of green tea extract loaded into nanostructured lipid carriers. Comptes Rendus Chimie, 2014. 17(4): p. 331-341.

16. Mahdavi, J., et al., Helicobacter pylori SabA adhesin in persistent infection and chronic inflammation. Science, 2002. 297(5581): p. 573-8.

17. Neves, A.R., et al., Novel resveratrol nanodelivery systems based on lipid nanoparticles to enhance its oral bioavailability. Int J Nanomedicine, 2013. 8: p. 177-87.

18. Lin, Y.H., et al., Development of pH-responsive chitosan/heparin nanoparticles for stomach-specific anti-Helicobacter pylori therapy. Biomaterials, 2009. 30(19): p. 3332-42.

19. Fonte, P., et al., Chapter fifteen - Chitosan-Coated Solid Lipid Nanoparticles for Insulin Delivery, in Methods in Enzymology, D. Nejat, Editor. 2012, Academic Press. p. 295-314.

20. Clogston, J. and A. Patri, Zeta Potential Measurement, in Characterization of Nanoparticles Intended for Drug Delivery, S.E. McNeil, Editor. 2011, Humana Press. p. 63-70.

21. Matuschek, E., D.F.J. Brown, and G. Kahlmeter, Development of the EUCAST disk diffusion antimicrobial susceptibility testing method and its implementation in routine microbiology laboratories. Clinical Microbiology and Infection, 2014. 20: p. O255-O266.

22. Kusters, J.G., A.H.M. van Vliet, and E.J. Kuipers, Pathogenesis of Helicobacter pylori Infection. Clinical Microbiology Reviews, 2006. 19(3): p. 449-490.

23. Hemarajata, P. and J. Versalovic, Effects of probiotics on gut microbiota: mechanisms of intestinal immunomodulation and neuromodulation. Therap Adv Gastroenterol, 2013. 6(1): p. 39-51.

24. Okuro, P.K., et al., Co- encapsulation of Lactobacillus acidophilus with inulin or polydextrose in solid lipid microparticles provides protection and improves stability. Food Research International, 2013. 53(1): p. 96-103.

25. Allocati, N., et al., Escherichia coli in Europe: an overview. Int J Environ Res Public Health, 2013. 10(12): p. 6235-54.

26. Reshes, G., et al., Cell shape dynamics in Escherichia coli. Biophys J, 2008. 94(1): p. 251-64.

27. Blanchard, T.G. and J.G. Nedrud, Laboratory Maintenance of Helicobacter Species. Current Protocols in Microbiology, 2006. CHAPTER: p. Unit8B.1-Unit8B.1.

## Claims

1. A nanostructured lipid particle for use in the treatment or prevention of a disease induced by Helicobacter pylori comprising

   a mixture of a solid lipid, a liquid lipid, a surfactant;
   wherein the solid lipid is glyceryl palmitostearate;
   wherein the liquid lipid is Caprylic/Capric Triglyceride;
   wherein the surfactant is polysorbate 60;
   wherein the nanostructured lipid particle is a Helicobacter pylori bactericide.

2. The nanostructured lipid particle for use according to any one of the previous claims, wherein the particles are obtainable by mixture of several lipids forming a nanoemulsion, with hot homogenization and ultrasonication.

3. The nanostructured lipid particle for use according to any one of the previous claims, wherein the particles diameters is between 50 and 400 nanometers, preferably between 150 and 250 nanometers.

4. The nanostructured lipid particle for use according to any one of the previous claims for storage during 1 month, in aqueous suspension at 4° - 20 °C.

5. An oral composition for use in the treatment or prevention of a disease induced by Helicobacter pylori comprising the nanostructured lipid particle described in any one of the previous claims in a effective amount.

6. Composition for use according to the previous claim further comprising at least one therapeutically active substance selected from the group consisting of a gastrointestinal protectant or an antibiotic, or mixtures thereof.

7. Composition for use according to the previous claim wherein the gastrointestinal protectants is a proton pump inhibitor.

8. Composition for use according to any one of the previous claims wherein the antibiotic is selected from a group consisting of: amoxicillin, clarithromycin, metronidazole, tetracycline, or mixtures thereof.

9. Composition for use according to any one of the previous claims further comprising gastric retentive polymer such us chitosan.

## Patentansprüche

1. Ein nanostrukturiertes Lipidpartikel zur Verwendung bei der Behandlung oder der Vorbeugung einer durch Helicobacter pylori induzierten Krankheit, umfassend

> eine Mischung aus einem festen Lipid, einem flüssigen Lipid und einem Tensid;
> wobei das feste Lipid Glycerylpalmitostearat ist;
> wobei das flüssige Lipid Caprylic/Capric Triglycerid ist;
> wobei das Tensid Polysorbat 60 ist;
> wobei der nanostrukturierte Lipidpartikel ein Helicobacter pylori Bakterizid ist.

2. Der nanostrukturierte Lipidpartikel zur Verwendung nach einem der vorangehenden Ansprüche, wobei die Partikel durch Mischen mehrerer Lipide, die eine Nanoemulsion bilden, durch Heißhomogenisierung und Ultraschallbehandlung gewonnen werden können.

3. Der nanostrukturierte Lipidpartikel zur Verwendung nach einem der vorangehenden Ansprüche, wobei der Partikeldurchmesser zwischen 50 und 400 Nanometern, bevorzugt zwischen 150 und 250 Nanometern liegt.

4. Der nanostrukturierte Lipidpartikel zur Verwendung nach einem der vorangehenden Ansprüche zur Lagerung während 1 Monat in einer wässrigen Suspension bei 4° - 20 °C.

5. Eine orale Zusammensetzung zur Verwendung bei der Behandlung oder der Vorbeugung einer durch Helicobacter pylori induzierten Krankheit, umfassend das in einem der vorangehenden Ansprüche beschriebene nanostrukturierte Lipidpartikel in einer wirksamen Menge.

6. Zusammensetzung zur Verwendung nach dem vorangehenden Anspruch ferner umfassend mindestens eine therapeutisch wirksame Substanz, die aus einer Gruppe ausgewählt ist, umfassend ein gastrointestinales Schutzmittel oder ein Antibiotikum oder Mischungen davon.

7. Zusammensetzung zur Verwendung nach dem vorangehenden Anspruch, wobei das gastrointestinale Schutzmittel ein Protonenpumpen-Inhibitor ist.

8. Zusammensetzung zur Verwendung nach einem der vorangehenden Ansprüche, wobei das Antibiotikum ausgewählt ist aus einer Gruppe umfassend: Amoxicillin, Clarithromycin, Metronidazol, Tetracyclin oder Mischungen davon.

9. Zusammensetzung zur Verwendung nach einem der vorangehenden Ansprüche ferner umfassend ein magensaftresistentes Polymer wie Chitosan.

**Revendications**

1. Une particule lipidique nanostructurée destinée à être utilisée dans le traitement ou la prévention d'une maladie induite par Helicobacter pylori comprenant

> un mélange d'un lipide solide, d'un lipide liquide et d'un tensioactif ;
> dans lequel le lipide solide est le palmitostéarate de glycéryle ;
> dans lequel le lipide liquide est le triglycéride caprylique/caprique ;
> dans lequel le tensioactif est le polysorbate 60 ;
> dans lequel la particule lipidique nanostructurée est un bactéricide Helicobacter pylori.

2. La particule lipidique nanostructurée destinée à être utilisée selon l'une quelconque des revendications précédentes, dans laquelle les particules peuvent être obtenues par mélange de plusieurs lipides formant une nanoémulsion, avec homogénéisation à chaud et ultrasonication.

3. La particule lipidique nanostructurée destinée à être utilisée selon l'une quelconque des revendications précédentes, dans laquelle le diamètre des particules est compris entre 50 et 400 nanomètres, de préférence entre 150 et 250 nanomètres.

4. La particule lipidique nanostructurée destinée à être utilisée selon l'une quelconque des revendications précédentes, pour être stockée pendant 1 mois, en suspension aqueuse à 4° - 20 °C.

**5.** Une composition orale destinée à être utilisée dans le traitement ou la prévention d'une maladie induite par Helicobacter pylori comprenant la particule lipidique nanostructurée décrite dans l'une quelconque des revendications précédentes dans une quantité effective.

**6.** Composition destinée à être utilisée selon la revendication précédente comprenant en outre au moins une substance thérapeutiquement active choisie dans le groupe constitué par un agent protecteur gastro-intestinal ou un antibiotique, ou des mélanges de ceux-ci.

**7.** Composition destinée à être utilisée selon la revendication précédente dans laquelle l'agent protecteur gastro-intestinal est un inhibiteur de la pompe à protons.

**8.** Composition destinée à être utilisée selon l'une quelconque des revendications précédentes, dans laquelle l'antibiotique est choisi dans un groupe constitué par : l'amoxicilline, la clarithromycine, le métronidazole, la tétracycline, ou des mélanges de ceux-ci.

**9.** Composition destinée à être utilisée selon l'une quelconque des revendications précédentes, comprenant en outre un polymère à rétention gastrique tel que le chitosane.

**A**

| | |
|---|---|
| Diameter (nm) | 211 ± 8 |
| Polydispersion index | 0.2±0.02 |
| ξ-potential (mV) | -28 ± 3 |

**Fig. 1 A-B**

**Fig. 1 C**

Fig. 2- A

Fig. 2- B

**Fig. 2- C**

**Fig. 2- D**

Fig. 2- E

Fig. 3-A

Fig. 3-B

Fig. 4

Fig. 5

Fig. 6-A

Fig. 6-B

## REFERENCES CITED IN THE DESCRIPTION

### Patent documents cited in the description

- US 20130017239 A1, Viladot Petit, J.L.L., G.R. Delgado, and B.A. Fernández

### Non-patent literature cited in the description

- **GONÇALVES et al.** *Expert review of anti-infective therapy*, 2014, vol. 12 (8)
- **CORREA, P.** ; **J. HOUGHTON**. Carcinogenesis of Helicobacter pylori.. *Gastroenterology*, 2007, vol. 133 (2), 659-672
- **AMIEVA, M.** ; **R.M. PEEK JR**. Pathobiology of Helicobacter pylori-Induced Gastric Cancer. *Gastroenterology*, 2016, vol. 150 (1), 64-78
- **MATYSIAK-BUDNIK, T.** ; **F. MÉGRAUD**. Helicobacter pylori infection and gastric cancer. *. European Journal of Cancer*, 2006, vol. 42 (6), 708-716
- **MALFERTHEINER, P. et al.** Management of Helicobacter pylori infection-the Maastricht IV/ Florence Consensus Report.. *Gut*, 2012, vol. 61 (5), 646-664
- **VAKIL, N.** Helicobacter pylori treatment: a practical approach.. *Am J Gastroenterol*, 2006, vol. 101 (3), 497-9
- **CARBONE, C. et al.** Pharmaceutical and biomedical applications of lipid-based nanocarriers. *Pharm Pat Anal*, 2014, vol. 3 (2), 199-215
- **BATTAGLIA, L** ; **M. GALLARATE**. Lipid nanoparticles: state of the art, new preparation methods and challenges in drug delivery. *Expert Opinion on Drug Delivery*, 2012, vol. 9 (5), 497-508
- **MARTINS, S. et al.** Lipid-based colloidal carriers for peptide and protein deliveryliposomes versus lipid nanoparticles. *Int J Nanomedicine*, 2007, vol. 2 (4), 595-607
- **MÜLLER, R.H.** ; **K. MÄDER** ; **S. GOHLA**. Solid lipid nanoparticles (SLN) for controlled drug delivery - a review of the state of the art.. *European Journal of Pharmaceutics and Biopharmaceutics*, 2000, vol. 50 (1), 161-177
- **MEHNERT, W.** ; **K. MÄDER**. Solid lipid nanoparticles: Production, characterization and applications.. *Advanced Drug Delivery Reviews*, 2001, vol. 47 (2-3), 165-196
- Solid lipid nanoparticles - a promising drug delivery system. **SVILENOV, H.T.** ; **CHRISTO**. Nanomedicine. One Central Press (OCP), 2014
- **MUCHOW, M.** ; **P. MAINCENT** ; **R.H. MÜLLER**. Lipid Nanoparticles with a Solid Matrix (SLN®, NLC®, LDC®) for Oral Drug Delivery. *Drug Development and Industrial Pharmacy*, 2008, vol. 34 (12), 1394-1405
- **DA SILVA, P.B et al.** A Nanostructured Lipid System as a Strategy to Improve the in Vitro Antibacterial Activity of Copper(II) Complexes.. *Molecules*, 2015, vol. 20 (12), 22534-45
- **MANEA, A.-M.** ; **B.S. VASILE** ; **A. MEGHEA**. Antioxidant and antimicrobial activities of green tea extract loaded into nanostructured lipid carriers.. *Comptes Rendus Chimie*, 2014, vol. 17 (4), 331-341
- **MAHDAVI, J. et al.** Helicobacter pylori SabA adhesin in persistent infection and chronic inflammation. *Science*, 2002, vol. 297 (5581), 573-8
- **NEVES, A.R. et al.** Novel resveratrol nanodelivery systems based on lipid nanoparticles to enhance its oral bioavailability.. *Int J Nanomedicine*, 2013, vol. 8, 177-87
- **LIN, Y.H. et al.** Development of pH-responsive chitosan/heparin nanoparticles for stomach-specific anti-Helicobacter pylori therapy.. *Biomaterials*, 2009, vol. 30 (19), 3332-42
- Chapter fifteen - Chitosan-Coated Solid Lipid Nanoparticles for Insulin Delivery. **FONTE, P. et al.** Methods in Enzymology. Academic Press, 2012, 295-314
- **CLOGSTON, J.** ; **A. PATRI**. Zeta Potential Measurement, in Characterization of Nanoparticles Intended for Drug Delivery. Humana Press, 2011, 63-70
- **MATUSCHEK, E.** ; **D.F.J. BROWN** ; **G. KAHLMETER**. Development of the EUCAST disk diffusion antimicrobial susceptibility testing method and its implementation in routine microbiology laboratories.. *Clinical Microbiology and Infection*, 2014, vol. 20
- **KUSTERS, J.G.** ; **A.H.M. VAN VLIET** ; **E.J. KUIPERS**. Pathogenesis of Helicobacter pylori Infection.. *Clinical Microbiology Reviews*, 2006, vol. 19 (3), 449-490

- **HEMARAJATA, P.** ; **J. VERSALOVIC**. Effects of probiotics on gut microbiota: mechanisms of intestinal immunomodulation and neuromodulation. *Therap Adv Gastroenterol*, 2013, vol. 6 (1), 39-51
- **OKURO, P.K. et al.** Co- encapsulation of Lactobacillus acidophilus with inulin or polydextrose in solid lipid microparticles provides protection and improves stability.. *Food Research International*, 2013, vol. 53 (1), 96-103
- **ALLOCATI, N. et al.** Escherichia coli in Europe: an overview.. *Int J Environ Res Public Health*, 2013, vol. 10 (12), 6235-54
- **RESHES, G. et al.** Cell shape dynamics in Escherichia coli.. *Biophys J*, 2008, vol. 94 (1), 251-64
- **BLANCHARD, T.G.** ; **J.G. NEDRUD**. Laboratory Maintenance of Helicobacter Species.. *Current Protocols in Microbiology*, 2006